# EUROPEAN PATENT APPLICATION

(11) **EP 2 075 276 A1**
(43) Date of publication of application: **01.07.2009**
(21) Application number: 06836011.4
(22) Date of filing: 30.10.2006
(51) Int. Cl.: C08G 77/14, A61P 39/00

(54) **METHOD FOR PRODUCING A SORBENT BASED ON A METHYL- SILICIC ACID HYDROGEL**

(30) Priority: 20.09.2006 UA 2006010083
(71) Applicant: Zakryte Aktsionerne Tovarystvo "Ekologoohoronna" Firma "Kreoma-Farm", Kiev 03680 (UA)
(72) Inventor: TOLCHEYEV, Yuriy, Kiev, 04210 (UA); CHYGYRYK, Oleksandr, Kiev, 02154 (UA); SEMENOV, Volodymyr, Kiev, 02160 (UA)
(74) Representative: Andrae, Steffen
(86) International application number: PCT/UA2006/000057
(87) International publication number: WO 2008/036056

(57) **Abstract**

The inventive method for producing a sorbent consists in adding a strong acid solution to a methylsiliconate sodium (or potassium) solution for obtaining a product, which after being stabilised, is ground and activated by adding a strong acid to the solution, is washed out by water until a neutral reaction is attained in such a way that a product, the pore space of which is equal to or greater that 0.8 cm³/g is obtained, wherein said product exhibits adsorption properties with respect to mean- and high-molecular agents and can contain chemical and/or biological additives. The thus obtained product has a general formula: {(CH₃SiO_{1,5})nH₂O}[k₁...Kₘ], wherein ki is a mass ratio (mass%) for chemical additives (m≥2) or a qualitative characteristic for the content of biological additives; xerogel is produced at n=0; gel form is produced at n=30-46; paste form is produced at n=54-62; suspension form is produced at n=63-495.

## Description

### Technical Field

The method for producing a sorbent based on a methylsilicic acid hydrogel relates to the field of pharmaceutical chemistry, particularly to a method of preparing new chemical compounds, namely, polymethylsiloxanes/methylsilicic acid hydrogels, and can find use in various domains of medicine and veterinary science as a sorbent for an excretion from an organism of toxic mean- and high-molecular products of metabolism that are accumulated in the organism when in various diseases and intoxications.

### Background Art

Adsorption of toxic mean-molecular metabolites by the novel product eliminates endotoxicosis, which develops with many diseases including infectious, inflammatory, autoimmune diseases, cancer and other diseases.

However, the obtained product is the most active in sorbing high-molecular substances, which include the microbial endotoxin, specific toxins of bacteria and a large number of regulatory proteins. An endotoxin bounded by a sorbent becomes inactive and is excreted together with the sorbent by natural ways. An antigenic load on immunocompetent cells is thereby reduced to reduce in its turn the intensity of autoimmune reactions and to contribute to compensation of immunodeficiency (secondary), which arises as a result of a pathologic process.

Adsorption of a series of regulatory proteins (enzymes, cytotoxins, immunoglobulins) by the novel product accounts for distant effects of enterosorption, i.e., normalization of viscera and systems, lipid spectrum of blood plasma, thereby contributing to diminishing severity of diseases, preventing development of multiple organ failures and critical states.

Known in the art is a method for producing "ENTEROSGEL-SUPER" sorbent or a methylsilicic acid hydrogel (Ukrainian Patent No. 7472 entitled "ENTEROSGEL-SUPER methylsilicic acid hydrogels as adsorbents of mean-molecular metabolites and method of preparing thereof").

Features common to this invention and the prior art method are the following steps: adding a solution of a strong acid to a solution of sodium methylsiliconate or potassium methylsiliconate until a product is formed, then holding, comminuting, activating the product by adding a diluted solution of a strong acid and washing the product to a neutral reaction.

A disadvantage of the prior art method resides in the fact that the sorbent produced by the prior art method has an inefficient sorptive capacity with respect to high-molecular substances, nor various forms of the end product are contemplated as well to restrict the range of use of the product in medical practice and veterinary science.

### Disclosure of the Invention

The present invention aims to provide a method for producing a sorbent based on a methylsilicic acid hydrogel through the use of a certain concentration of sodium methylsiliconate or potassium methylsiliconate and various proportions of the product and water molecules as well as weight percentages of chemical and/or biological additives to ensure obtaining an end product featuring a high sorptive capacity.

The technical result of the present invention is the provision of a product of a high sorptive capacity with respect to both mean-molecular substances and high-molecular substances. The method also makes sure obtaining of various forms of the end product.

To the accomplishment of the foregoing object, there is provided a method for producing a sorbent based on a methylsilicic acid hydrogel of the general formula

**{(CH₃SiO_{1.5})_{∞}·nH₂O},**

comprising the following steps: adding a solution of a strong acid to a solution of sodium methylsiliconate or potassium methylsiliconate until a product is formed, then holding, comminuting, activating the product by adding a diluted solution of a strong acid and washing the product to a neutral reaction, wherein, according to the invention, the solution of sodium methylsiliconate or potassium methylsiliconate is at a concentration providing for obtaining a product with a pore volume of at least 0.8 cm³/g and having adsorption properties with respect to both mean-molecular substances and high-molecular substances, and can contain chemical and/or biological additives k.

The method will yield product of the general formula

**{(CH₃SiO_{1.5})_{∞}·nH₂O}· [k₁...kₘ],**

wherein kᵢ is percentage (weight %) of chemical additives (m≥2) or an amount of biological additives;
at n = 0, a product is obtained having a powdered form (xerogel) with additives (at kᵢ ≠ 0) or without additives (at kᵢ = 0);
at n = 30-46, the product has a gel-like form;
at n = 45-62, a product is obtained having a paste-like form with additives (at kᵢ ≠ 0) or without additives (at kᵢ = 0);
at n = 63-495, a product is obtained having a form of a suspension with additives (at kᵢ ≠ 0) or without additives (at kᵢ = 0).

According to the invention, in the method for producing a methylsilicic acid hydrogel, the solution of sodium methylsiliconate or potassium methylsiliconate is at a concentration of 2.30-2.95 mol/L.

According to the invention, a mixture of sweeteners at a concentration of 0.5 weight % is further added to the product.

According to the invention, copper sulfate or zinc sulfate at a concentration of 1.0 weight % is further added to the product.

According to the invention, preservatives at a concentration of 0.5 weight % are further added to the product.

According to the invention, eubiotics and/or probiotics are further added to the product at a concentration of 10⁶-10¹² CFU per gram of the product.

The method is carried out as follows.

A solution of sodium methylsiliconate or potassium methylsiliconate at a concentration of 2.35-2.95 mol/L is subjected to polycondensation by adding a solution of a strong acid (hydrochloric or sulfuric acid) until a hydrogel is formed. The hydrogel is held for 30-90 minutes until ripe, then comminuted, and activated under the action of a diluted solution of a strong acid at a concentration of 0.04-0.15 gram equivalents per liter and subsequently washing the product to a neutral reaction. Chemical or biological additives are further added. With changing the number of water molecules and the weight proportion of the chemical or biological additive, the obtained product may be in the form of a suspension or paste (following dispersing and subsequent homogenizing of a mixture), or the form of a powder (following drying of the product to constant mass), specifically:
at n = 0, a product is obtained having a powdered form (xerogel) with additives (at kᵢ ≠ 0) or without additives (at kᵢ = 0);
at n = 30-46, the product has a gel-like form;
at n = 45-62, a product is obtained having a paste-like form with additives (at kᵢ ≠ 0) or without additives (at kᵢ = 0);
at n = 63-495, a product is obtained having a form of a suspension with additives (at kᵢ ≠ 0) or without additives (at kᵢ = 0).

### Best Mode for Carrying Out the Invention

### Example 1 Method for producing a product

A solution of a strong acid is added to 100 mL of a solution of sodium methylsiliconate at the concentration of 2.30 mol/L until a hydrogel is formed. The hydrogel is held for some time until ripe, then comminuted, and the solution of the same strong acid, but diluted, is pored over the hydrogel. The resulting hydrogel is washed with water until it shows a negative reaction toward anions and has neutral acidity values.

### Examples 2-14

The hydrogel of methylsilicic acid is produced as in Example 1 and sodium methylsiliconate at a concentration of 2.35-2.95 mol/L is used as a starting reactant.

### Examples 15-16

The hydrogel of methylsilicic acid is produced as in Examples 1-14 and solutions of sodium methylsiliconate and of a strong acid (Example 15), and a mixture of solutions of sodium methylsiliconate and potassium methylsiliconate, and a strong acid (Example 16) are used as starting reactants.

### Example 17 Producing a paste-like product

Water in the amount of 30 g is added to 70 g of the methylsilicic acid hydrogel prepared as in Examples 1-16, and the hydrogel is dispersed and then homogenized until a paste-like product is formed.

### Examples 18-26 Producing a paste-like product with various proportions of the methylsilicic acid hydrogel and water

The methylsilicic acid hydrogel and water are mixed as in Example 17 taking 90 g methylsilicic acid hydrogel and 10 g water (Example 18), 85 g methylsilicic acid hydrogel and 15 g water (Example 19), 80 g methylsilicic acid hydrogel and 20 g water (Example 20), 75 g methylsilicic acid hydrogel and 25 g water (Example 21), 65 g methylsilicic acid hydrogel and 35 g water (Example 22), 60 g methylsilicic acid hydrogel and 40 g water (Example 23), 55 g methylsilicic acid hydrogel and 45 g water (Example 24), 50 g methylsilicic acid hydrogel and 50 g water (Example 25), 45 g methylsilicic acid hydrogel and 55 g water (Example 26).

### Example 27 Producing a suspension form of the product

Water in the amount of 70 g is added to 30 g of the methylsilicic acid hydrogel prepared as in Examples 1-16, and the hydrogel is dispersed and then homogenized until a paste-like product is formed.

### Examples 28-33 Producing a suspension form of the product with various proportions of the methylsilicic acid hydrogel and water

The methylsilicic acid hydrogel and water are mixed as in Example 27 taking 40 g methylsilicic acid hydrogel and 60 g water (Example 28), 35 g methylsilicic acid hydrogel and 65 g water (Example 29), 25 g methylsilicic acid hydrogel and 75 g water (Example 30), 20 g methylsilicic acid hydrogel and 80 g water (Example 31), 15 g methylsilicic acid hydrogel and 85 g water (Example 32), 10 g methylsilicic acid hydrogel and 90 g water (Example 33).

### Example 34 Producing the product with sweeteners

A mixture of sweeteners consisting of sodium cyclamate and saccharin is further added to 100 g of the product produced as in Examples 17-33. The amount of sweeteners in the end product is 0.2 weight %.

### Example 35 Producing the product with sucralose as a sweetener

The product is produced as in Example 34 and sucralose is used as a sweetener. The amount of sucralose in the end product is 0.1 weight %.

### Example 36 Producing the product with a chemical additive, which is sodium benzoate as a preservative

Sodium benzoate as a preservative is further added to 100 g of the product produced as in Examples 17-35. The amount of sodium benzoate in the end product is 0.5 weight %.

### Examples 37-40 Producing the product with various preservatives

The product is produced as in Example 36 and citric acid is used as a preservative, the content of which in the end product is 0.5 weight % (Example 37), tartaric acid is used as a preservative, the content of which in the end product is 0.5 weight % (Example 38), succinic acid is used as a preservative, the content of which in the end product is 0.5 weight % (Example 39), benzalkonium chloride is used as a preservative, the content of which in the end product is 0.5 weight % (Example 40).

### Example 41 Producing a powdery product

The product in the amount of 100 g prepared as in Examples 1-16 is dried at 125±5°C to constant mass. A xerogel in the amounts of 7.5 g to 11.0 g is produced.

### Example 42 A powdery product containing copper sulfate

The product is prepared as in Example 41 with copper sulfate previously added in the amount that is necessary for the copper sulfate content of 0.7 weight % to be in the end product.

### Example 43 A powdery product containing zinc sulfate

The product is prepared as in Example 41 with zinc sulfate previously added in the amount that is necessary for the zinc sulfate content of 0.7 weight % to be in the end product.

### Example 44 The product with biological additives, which are eubiotics

The product prepared as in Examples 17-26 is taken in the amount, based on the determination of a dry residue content, such that it may comprise 10 g of the dry residue and this amount is mixed with freeze-dried eubiotic cultures of *Bacillus subtilis* and *Bacillus licheniformis.* The amount of eubiotics is 10⁶-10¹² CFU per gram of the end product.

The present invention provides for obtaining a product of a high sorptive capacity with respect to both mean-molecular substances and high-molecular substances. The method also makes sure obtaining of various forms of the end product, namely, paste-like, powdery forms or in the form of a suspension.

Tables 1-3 show the properties of the products produced as described in the foregoing examples.

As seen in Table 1, relative sorptive capacities (as the ratio of the sorptive capacity with respect to high-molecular substances to the sorptive capacity with respect to mean-molecular substances) of the claimed products in the form of gels, pastes and suspensions are related as 1.0:1.5:2.0 respectively, to prove their discreteness and specificity.

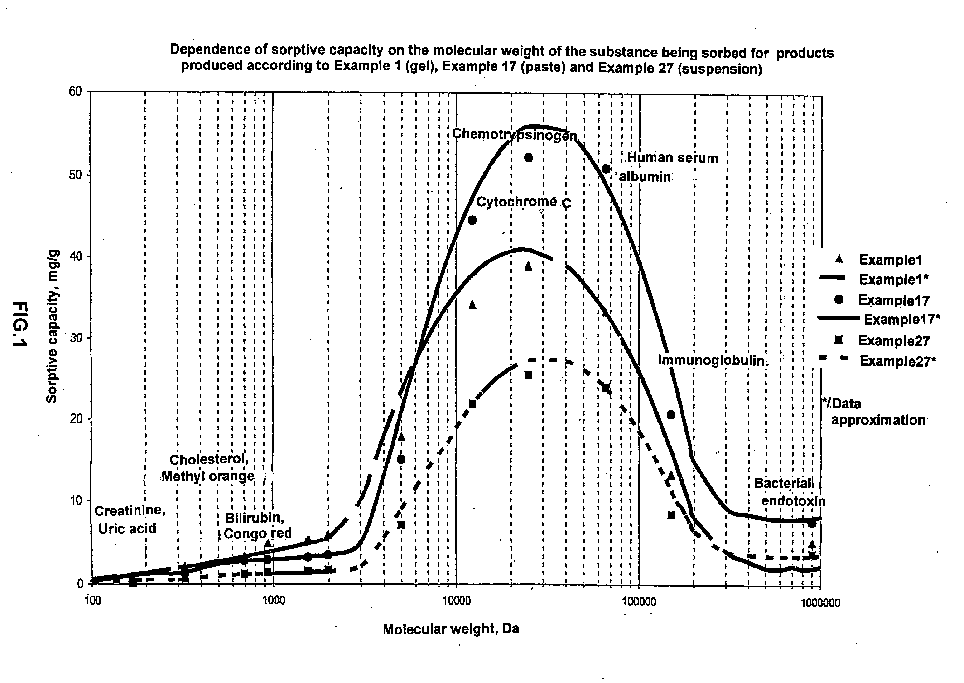

Some examples are represented in graphical form in Fig. 1.

In whole, the product produced according to the present invention is an effective detoxifier and at the same time it has a positive general regulatory effect on the organism to thereby enable patients to avoid complications and to make a fast recovery.

**Table 1**

| Properties of the products obtained according to Examples 1-33 (gel, paste, and suspension) | | | | | | |
|---|---|---|---|---|---|---|
| Example No. | Dry residue content, weight % | Silicon content, weight % | Sorptive capacity for Congo red, mg/g (α<0.01) | Sorptive capacity for human serum albumin, mg/g (α<0.01) | Ratio of sorptive capacities | Formula |
| 1 | 2 | 3 | 4 | 5 | 6 | 7 |
| 1.* | 11.0 | 4.75 | 3.3 | 33.4 | 10.1 | {CH₃SiO_{1.5}}·30H₂O |
| 2. | 11.0 | 4.75 | 3.3 | 33.2 | 10.1 | {CH₃SiO_{1.5}}·30H₂O |
| 3. | 10.4 | 4.48 | 3.3 | 33.1 | 10.0 | {CH₃SiO_{1.5}}·32H₂O |
| 4. | 9.9 | 4.25 | 3.1 | 31.7 | 10.2 | {CH₃SiO_{1.5}}·34H₂O |
| 5. | 9.4 | 4.03 | 3.0 | 30.2 | 10.0 | {CH₃SiO_{1.5}}·36H₂O |
| 6. | 8.9 | 3.84 | 2.8 | 28.2 | 10.1 | {CH₃SiO_{1.5}}·38H₂O |
| 7. | 8.5 | 3.66 | 2.7 | 27.5 | 10.2 | {CH₃SiO_{1.5}}·40H₂O |
| 8. | 8.2 | 3.50 | 2.6 | 26.6 | 10.2 | {CH₃SiO_{1.5}}·42H₂O |
| 9. | 7.8 | 3.36 | 2.5 | 25.3 | 10.1 | {CH₃SiO_{1.5}}·44H₂O |
| 10. | 7.5 | 3.22 | 2.4 | 24.4 | 10.2 | {CH₃SiO_{1.5}}·46H₂O |
| 11. | 9.6 | 4.14 | 3.0 | 30.8 | 10.3 | {CH₃SiO_{1.5}}·35H₂O |
| 12. | 9.2 | 3.93 | 2.9 | 30.0 | 10.4 | {CH₃SiO_{1.5}}·37H₂O |
| 13. | 7.6 | 3.29 | 2.4 | 24.7 | 10.3 | {CH₃SiO_{1.5}}·45H₂O |
| 14. | 8.3 | 3.58 | 2.7 | 26.9 | 10.0 | {CH₃SiO_{1.5}}·41H₂O |
| 15. | 10.1 | 4.36 | 3.2 | 32.3 | 10.1 | {CH₃SiO_{1.5}}·33H₂O |
| 16. | 8.0 | 3.43 | 2.6 | 26.0 | 10.0 | {CH₃SiO_{1.5}}·43H₂O |
| 17. * | 6.68 | 2.87 | 3.2 | 49.1 | 15.4 | {CH₃SiO_{1.5}}·52H₂O |
| 18. | 7.8 | 3.29 | 3.8 | 58.0 | 15.3 | {CH₃SiO_{1.5}}·45H₂O |
| 19. | 7.34 | 3.16 | 3.6 | 54.7 | 15.2 | {CH₃SiO_{1.5}}·47H₂O |
| 20. | 7.06 | 3.04 | 3.4 | 52.6 | 15.5 | {CH₃SiO_{1.5})-49H₂O |
| 21. | 6.93 | 2.98 | 3.4 | 51.6 | 15.2 | {CH₃SiO_{1.5}}·50H₂O |
| 22 | 6.57 | 2.82 | 3.2 | 49.0 | 15.3 | {CH₃SiO_{1.5}}·53H₂O |
| 23. | 6.34 | 2.73 | 3.1 | 47.3 | 15.3 | {CH₃SiO_{1.5}}·55H₂O |
| 24. | 6.13 | 2.64 | 3.0 | 45.8 | 15.3 | {CH₃SiO_{1.5}}·57H₂O |
| 25. | 5.85 | 2.51 | 2.8 | 43.7 | 15.6 | {CH₃SiO_{1.5}}·60H₂O |
| 26. | 5.67 | 2.44 | 2.8 | 42.4 | 15.2 | {CH₃SiO_{1.5}}·62H₂O |
| 27. * | 3.74 | 1.61 | 1.2 | 24.0 | 20.0 | {CH₃SiO_{1.5}}·96H₂O |
| 28. | 5.58 | 2.40 | 1.8 | 36.1 | 20.1 | {CH₃SiO_{1.5}}·63H₂O |
| 29. | 4.73 | 2.03 | 1.5 | 30.6 | 20.4 | {CH₃SiO_{1.5}}·75H₂O |
| 30. | 2.68 | 1.15 | 0.85 | 17.5 | 20.6 | {CH₃SiO_{1.5}}·135H₂O |
| 31. | 1.99 | 0.86 | 0.65 | 13.1 | 20.2 | {CH₃SiO_{1.5}}·183H₂O |
| 32. | 1.35 | 0.58 | 0.45 | 9.0 | 20.0 | {CH₃SiO_{1.5}}·272H₂O |
| 33. | 0.75 | 0.32 | 0.25 | 5.1 | 20.4 | {CH₃SiO_{1.5}}·493H₂O |

| | | | | | | |
|---|---|---|---|---|---|---|
| * example represented in graphical form in Fig. 1. | | | | | | |

**Table 2**

| Properties of the products obtained according to Examples 34-40 and44 (with various additives) | | | | | | | |
|---|---|---|---|---|---|---|---|
| Example No. | Dry residue content, weight % | Silicon content, weight % | Sorptive capacity for Congo red, mg/g (α<0.01) | Sorptive capacity for human serum albumin, mg/g (α<0.01) | Ratio of sorptive capacities | Additive | Formula |
| 1 | 2 | 3 | 4 | 5 | 6 | | 7 |
| 34. | 6.57 | 2.81 | 3.2 | 49 | 15.3 | sodium cyclamate and saccharin | {CH₃SiO_{1.5}}·53H₂O· [C₇H₅NO₃S]·[C₆H₁₂O₃SNa] |
| 35. | 6.34 | 2.72 | 3.1 | 47.1 | 15.2 | sucralose | {CH₃SiO_{1.5}}·55H₂O· [C₁₂H₁₉O₈Cl₃] |
| 36. | 5.85 | 2.50 | 2.8 | 43.5 | 15.5 | sodium benzoate | {CH₃SiO_{1.5}}·60H₂O· [C₇H₅O₂Na]· |
| 37. | 4.73 | 2.02 | 1.5 | 30.4 | 20.3 | citric acid | {CH₃SiO_{1.5}}·75H₂O· [C₆H₈O₇] |
| 38. | 3.74 | 1.60 | 0.84 | 17.4 | 20.7 | tartaric acid | {CH₃SiO_{1.5}}·96H₂O· [C₄H₆O₆] |
| 39. | 2.68 | 1.14 | 1.2 | 23.9 | 19.9 | succinic acid | {CH₃SiO_{1.5}}·135H₂O· [C₄H₆O₄] |
| 40. | 1.35 | 0.58 | 0.45 | 9.0 | 20.0 | benzalkonium chloride | {CH₃SiO_{1.5}}·272H₂O. [C₂₁H₃₈CIN] |
| 44. | 6.93 | 2.95 | 3.4 | 51.1 | 15.0 | *Bacillus subtilis* and *Bacillus licheniformis* | {CH₃SiO_{1.5}}·50H₂O· [*Bacillus subtilis]* [*Bacillus licheniformis]* |

**Table 3**

| Properties of the products obtained according to Examples 41-43 (powder with various additives) | | | | | | |
|---|---|---|---|---|---|---|
| Example No. | Dry residue content, weight % | Silicon content, weight % | Pore sorption volume for hexane, cm³/g** | Surface area, m²/g** | Additive | Formula |
| 41. | 100 | 43.00 | 1.10 | 900 | - | {CH₃SiO_{1.5}} |
| 42. | 100 | 42.70 | 1.05 | 850 | copper sulfate | {CH₃SiO_{1.5}}·[CuSO₄] |
| 43. | 100 | 42.68 | 1.03 | 850 | zinc sulfate | {CH₃SiO_{1.5}}·[ZnSO₄] |

| | | | | | | |
|---|---|---|---|---|---|---|
| ** Determined by the BET (Brunauer-Emmett-Teller) method based on the multi-layer molecular adsorption model. | | | | | | |

## Claims

1. A method for producing a sorbent based on a methylsilicic acid hydrogel of the general formula
**{(CH₃SiO_{1.5})_{∞}·nH₂O},**
comprising the following steps:
adding a solution of a strong acid to a solution of sodium methylsiliconate or potassium methylsiliconate until a product is formed, then holding, comminuting, activating the product by adding a diluted solution of a strong acid and washing the product to a neutral reaction, **characterized in that** the solution of sodium methylsiliconate or potassium methylsiliconate is at a concentration providing for obtaining a product with a pore volume of at least 0.8 cm³/g and having adsorption properties with respect to both mean-molecular substances and high-molecular substances, and can contain chemical and/or biological additives k ,whereby it is obtained the product of the general formula
**{(CH₃SiO_{1.5})_{∞}·nH₂O}·[k₁...kₘ],**
wherein kᵢ is percentage (weight %) of chemical additives (m≥2) or an amount of biological additives;
at n = 0, a product is obtained having a powdered form (xerogel) with additives (at kᵢ ≠ 0) or without additives (at kᵢ = 0);
at n = 30-46, the product has a gel-like form;
at n = 45-62, a product is obtained having a paste-like form with additives (at kᵢ ≠ 0) or without additives (at kᵢ = 0);
at n = 63-495, a product is obtained having a form of a suspension with additives (at kᵢ ≠ 0) or without additives (at kᵢ = 0).

2. The method of claim 1, **characterized in that** the solution of sodium methylsiliconate or potassium methylsiliconate is at a concentration of 2.30-2.95 mol/L.

3. The method of claim 1, **characterized in that** a mixture of sweeteners at a concentration of 0.5 weight % is further added to the product.

4. The method of any one of claims 1- 3 , **characterized in that** preservatives at a concentration of 0.5 weight % are further added to the product.

5. The method of claim 1, **characterized in that** copper sulfate at a concentration of 1.0 weight % is further added to the product.

6. The method of claim 1, **characterized in that** zinc sulfate at a concentration of 1.0 weight % is further added to the product.

7. The method of claim 1, **characterized in that** eubiotics and/or probiotics are further added to the product at a concentration of 10⁶-10¹² CFU per gram of the product.

## Amended claims

### Amended claims under Art. 19.1 PCT

**1.** Method for producing a sorbent based on a methylsilicic acid hydrogel of the general formula
**{(CH₃SiO_{1.5})_{∞}**·**nH₂O},**
comprising the following steps: adding a solution of a strong acid to a solution of sodium methylsiliconate or potassium methylsiliconate until a product is formed, then holding, comminuting, activating the product by adding a diluted solution of a strong acid and washing the product to a neutral reaction, **characterized in that** the solution of sodium methylsiliconate or potassium methylsiliconate is at a concentration of 2.35-2.95 mol/L and, by varying the multiplier n, the sorbent is obtained in a powdered, gel or paste-like form and exhibiting selective adsorption properties with respect to high-molecular substances with a molecular weight of 10,000-500,000 Dalton and more.

**2.** The method of claim 1, **characterized in that** the obtained sorbent is mixed with additives to form an end product of the general formula
**{(CH₃SiO_{1.5})_{∞}·nH₂O}·[k₁...kₘ],**
wherein kᵢ is percentage (weight %) of chemical additives (m≥2) or an amount of biological additives.

**3.** The method of claim 2, **characterized in that** the additives are a mixture of sweeteners at a concentration of 0.5 weight %.

**4.** The method of claim 2, **characterized in that** the additives are a mixture of sweeteners at a concentration of 0.5 weight %.

**5.** The method of claim 2, **characterized in that** the additives are preservatives at a concentration of 1.0 weight %.

**6.** The method of claim 2, **characterized in that** the additives include zinc sulfate at a concentration of 1.0 weight %.

**7.** The method of claim 2, **characterized in that** the additives are eubiotics and/or probiotics at a concentration of 10⁶-10¹² CFU per gram of the end product.
